# EUROPEAN PATENT APPLICATION

(11) **EP 4 729 941 A1**
(43) Date of publication of application: **22.04.2026**
(21) Application number: 24216062.0
(22) Date of filing: 28.11.2024
(51) Int. Cl.: G01N 33/543, G01N 33/44, G01N 33/548

(54) **ONE-STEP PRECIPITATION AND COLOR DEVELOPMENT DETECTION METHOD OF NITROCELLULOSE (NC) MEMBRANE FOR MICRO/NANO PLASTICS**

(30) Priority: 15.10.2024 CN 202411439583
(71) Applicant: Wuhan Textile University, Wuhan City, Hubei 430000 (CN)
(72) Inventor: Zhang, Zhaowei, Wuhan City (CN); Wu, Wenqin, Wuhan City (CN)
(74) Representative: Cleanthous, Marinos

(57) **Abstract**

A one-step precipitation and color development detection method of a nitrocellulose (NC) membrane for micro/nano plastics was provided, including the following steps: S1, preparing a micro/nano plastic-ovalbumin (OVA) conjugate; S2, preparing an NC membrane coated with the micro/nano plastic-OVA conjugate; S3, putting the NC membrane coated with the micro/nano plastic-OVA conjugate into a polystyrene antibody solution and a sample solution and incubating for 1 h at 37°C, and then rinsing the NC membrane; S4, diluting goat anti-mouse IgG-horseradish peroxidase (HRP) by 1w fold and then putting in a centrifuge tube, putting the NC membrane into the centrifuge tube, and then incubating for 30 min at 37°C; and S5, washing the NC membrane 4-5 times, completely immersing the NC membrane into a precipitating tetramethylbenzidine (TMB) for 30 s and then taking out the NC membrane at room temperature for development, followed by detecting the concentration of micro/nano plastics.

## Description

### TECHNICAL FIELD

The present disclosure relates to a one-step precipitation and color development detection method of a nitrocellulose (NC) membrane for micro/nano plastics.

### BACKGROUND

Microplastics are a new type of environmental pollutants, which can adsorb heavy metals and persistent organic pollutants, and migrate and transform in the environment. The microplastics are easily ingested by animals and toxic and harmful substances carried by the microplastics are released, thereby causing damage to the growth, development, reproduction, gene expression, and other aspects of living organisms.

At present, the quantitative analysis methods for microplastics mainly include a microscopic infrared spectroscopy method, a microscopic Raman spectroscopy method, a visual inspection method, etc. The visual inspection method is to count the number of microplastic particles by manually counting, and picking out and weighing all of the microplastics, which is not only time-consuming and laborious but also prone to errors during operation. The microscopic infrared spectroscopy method and the microscopic Raman spectroscopy method are the same as the visual inspection method in a quantifying manner, but they recognize particles instead of the naked eye, which greatly improves the accuracy of analysis. The microplastics must be identified one by one if the microscopic infrared spectroscopy method and the microscopic Raman spectroscopy method are used, and the surface of the identified microplastics cannot be polluted by organic pollutants, which needs a good pretreatment technology and a large amount of analysis time.

Therefore, it is urgent to provide a one-step precipitation and color development method of an NC membrane for micro/nano plastics.

### SUMMARY

To solve the defects existing in the prior art, the present disclosure provides a one-step precipitation and color development method of an NC membrane for micro/nano plastics.

To solve the above technical problem, the present disclosure provides the following technical solution:
The present disclosure provides a one-step precipitation and color development method of an NC membrane for micro/nano plastics, comprising the following steps:
S1, coupling micro/nano plastics with ovalbumin (OVA) to prepare a micro/nano plastic-OVA conjugate;
S2, streaking the micro/nano plastic-OVA conjugate on an NC membrane to prepare an NC membrane coated with the micro/nano plastic-OVA conjugate;
S3, putting the NC membrane coated with the micro/nano plastic-OVA conjugate into a polystyrene antibody solution and a sample solution to be incubated for 1 h at 37°C, and then rinsing the NC membrane once with phosphate-buffered saline Tween (PBST) and taking out the rinsed NC membrane; wherein the polystyrene antibody is secreted by hybridoma cell line polystyrene (PS)-8 which is deposited with China Center for Type Culture Collection under the Accession number of CCTCC NO: C2024144 on May 10, 2024, and the address of the collection unit is 299 Bayi Road, Wuchang District, Wuhan City, Hubei Province, on campus of Wuhan University;
S4, diluting goat anti-mouse lgG-horseradish peroxidase (HRP) with 20% N-bromosuccinimide-phosphate buffer saline (NBS-PBS) by 1w fold and then putting the diluted goat anti-mouse IgG-HRP in a centrifuge tube, putting the NC membrane into the centrifuge tube, and then incubating for 30 min at 37°C; and
S5, washing the NC membrane 4-5 times and sucking away water, completely immersing the NC membrane into precipitating tetramethylbenzidine (TMB) for 30 s and then taking out the NC membrane and sucking away water, and placing the NC membrane at room temperature for color development, followed by detecting the concentration of micro/nano plastics.

Preferably, in step S1, the coupling of the micro/nano plastics with OVA comprises the following steps:
S11, cooling the microplastics, polylinker coupling buffer, and polylinker wash/storage buffer to room temperature;
S12, putting the microplastics into a centrifuge tube, sucking away the supernatant after centrifuging, adding the polylinker coupling buffer for resuspension, sucking away the supernatant after centrifuging again, and resuspending with the polylinker coupling buffer;
S13, dissolving polylinker ethyl-[3-(dimethylamino)propyl]-carbodiimide hydrochloride (EDAC) into the polylinker coupling buffer to prepare an EDAC solution;
S14, adding the EDAC solution into the microplastic suspension, and then evenly mixing the above materials for activation;
S15, adding an OVA protein, diluting the OVA protein into 1-5 mg/mL with the polylinker coupling buffer, and evenly mixing the above materials for 30-60 min under the condition of rotating at room temperature;
S16, sucking away the supernatant after centrifuging;
S17, adding 0.4 mL of polylinker coupling buffer for resuspension; and
S18, repeating steps S16 and S17 to finally obtain the micro/nano plastic-OVA conjugate, and then storing the conjugate at 4°C.

Preferably, in step S5, detecting the concentration of the micro/nano plastics comprises the following steps:
S51, photographing the NC membrane to obtain the color developed with precipitating tetramethylbenzidine (TMB);
S52, transforming the color developed with the precipitating TMB into a gray value and then establishing a standard curve with the transformed gray value and the concentration; and
S53, calculating the concentration of the micro/nano plastics in a to-be-detected sample by inputting the gray value of the to-be-detected sample into the standard curve.

Compared with the prior art, the present disclosure has the beneficial effects:
The NC membrane of the present disclosure can be directly detected in a solution (wet-process detection) without a sample pad and a water adsorption pad, thereby saving cost; meanwhile, visualized, qualitative, and quantitative detection is achieved without freeze-dried powders and electrophoresis gel.

### Collection of cells:

The polystyrene antibody provided by the present disclosure is secreted by hybridoma cell line PS-8 which was deposited with the China Center for Type Culture Collection under the Accession numbers of CCTCC NO: C2024144 on May 10, 2024, and the address of the collection unit is 299 Bayi Road, Wuchang District, Wuhan City, Hubei Province, on the campus of Wuhan University.

### BRIEF DESCRIPTION OF THE DRAWINGS

FIG. 1 is a structural diagram of one-step precipitating and developing test paper for a microplastic NC membrane in the present disclosure;
FIG. 2 shows a result criterion of one-step precipitating and developing detection for a microplastic NC membrane in the present disclosure; and
FIG. 3 shows the results of one-step precipitating and developing detection for a microplastic NC membrane in the present disclosure.

### DETAILED DESCRIPTION OF THE EMBODIMENTS

Next, the preferred embodiments of the present disclosure will be illustrated in combination with accompanying drawings, it should be understood that the preferred embodiments described here are only for illustrating and explaining the present disclosure, but not limiting the present disclosure.

### Example 1: Coupling of microplastic polystyrene, carboxyl (COOH) (PS-CHOOH) and OVA

1. Microplastics, polylink coupling buffer, and polylink wash/storage buffer were cooled to room temperature.
2. 12.5 mg of microplastics were added into a 1.5 mL centrifuge tube and then centrifuged for 5 min at 500-1000 g, and supernatant was carefully sucked away; 0.4 mL of polylink coupling buffer was added for resuspension and then the suspension was centrifuged for 5 min at 500-1000 xg, the supernatant was carefully sucked away, and 0.17 mL of polylink coupling buffer was added for resuspension.
3. 10 mg of polylink EDAC was dissolved into 50 µL of polylink coupling buffer to prepare a 200 mg/mL EDAC solution. The solution was prepared when in use.
4. 20 µL of EDAC solution was added into a microplastic suspension to be evenly mixed for 15 min for activation.
5. 500 µg of OVA protein was added and diluted into 5 mg/mL using the polylink coupling buffer, and then the above materials were evenly mixed for 30 min under the condition of rotating at room temperature.
6. The obtained mixed solution was centrifuged for 10 min at 500-1000 xg, and then the supernatant was sucked away.
7. 0.4 mL of polylinker wash/storage buffer was added for resuspension, steps 6 and 7 were repeated to obtain PS-OVA, and finally PS-OVA was stored at 4°C.
8. Sodium dodecyl sulfate-polyacrylamide gel electrophoresis (SDS-PAGE) analysis (12% gel) was performed.

### Example 2: NC membrane was coated with PS-OVA

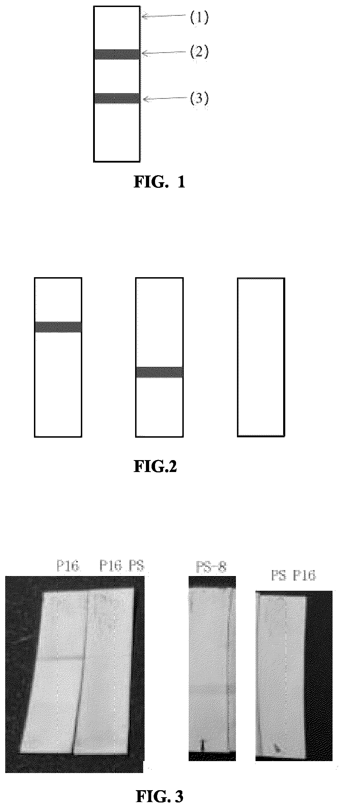

1. 30 µL of PS-OVA was streaked on an NC membrane using a test strip scribing instrument in a spraying amount of 0.08 µL/cm, and 30 µL of 1 mg/mL p16 protein was taken as control to be streaked on another NC membrane. The NC membrane is shown in FIG. 1; the p16 protein is a cyclin-dependent kinase inhibitor as positive control and negative control.
2. The NC membrane was placed at a humidity of less than 15% and dried at 50°C overnight.

### Example 3: Detection of NC membrane

1. The PS-8 monoclonal antibody was diluted into 2 µg/mL using 20% NBS-PBS, and 2 mL of antibody solution was put in a 5 mL centrifuge tube; the control monoclonal antibody p16-25 was diluted into 2 µg/mL using 20% NBS-PBS, and 2 mL of antibody solution was put in the 5 mL centrifuge tube.
2. The NC membrane coated with PS-OVA was cut into small sections with the same width, and then respectively put in the PS-8 antibody solution and the p16-25 control antibody solution (as negative control); the NC membrane coated with p16 was cut into small sections with the same width and then put in the p16-25 control antibody solution, and subsequently another 1 cut section was put in a PS-1 antibody solution as negative control; a PS sample extracting dilution solution was added into the antibody solutions.
3. The NC membranes were incubated for 1 h at 37°C and rinsed once with PBST.
4. Goat anti-mouse 1gG-HRP was subjected to 1 w dilution with 20% NBS-PBS, 10 mL of diluted anti-mouse 1gG-HRP, and the NC membranes were respectively put in a centrifuge tube, and then incubated for 30 min at 37°C.
5. The NC membranes were washed 4-5 times, water was sucked away with paper, the NC membranes were completely immersed into precipitating TMB for 30 s and then taken out, water was sucked away with paper, the NC membranes were placed at room temperature for color development, and then the results were photographed by intelligent detection equipment in a mobile phone.
6. A standard curve was established according to precipitating TMB developing color (transformed into a gray value) by an intelligent detection application (APP), and the concentration of micro/nano plastics in a to-be-detected sample was calculated by inputting the test gray value of the to-be-detected sample into the standard curve. As shown in FIG. 2, the result standards, from left to right, are negative, positive, and invalid. The results tested in this example are shown in FIG. 3.

### Example 4: Preparation of PS-8 monoclonal antibody

(1) Coupling of microplastic (PS-CHOOH) and coupling protein (BSA/keyhole limpet hemocyanin (KLH)): microplastics, polylink coupling buffer, polylink EDAC, and polylink wash/storage buffer were cooled to room temperature. 12.5 mg of microplastics were added into a 1.5 mL centrifuge tube and then centrifuged for 5-10 min at 500-1000 g, and the supernatant was discarded; 0.4 mL of polylink coupling buffer was added for resuspension followed by centrifugation again, and then the supernatant was discarded; 0.17 mL of polylink coupling buffer was added again for resuspension. 10 mg of polylink EDAC was dissolved into 50 µL of polylink coupling buffer to prepare a 200 mg/mL EDAC solution. Note: the solution was prepared when using. 20 µL of EDAC solution was added into 0.17 mL of polylink coupling buffer suspension to be fixed on a rotator to be evenly mixed for 15 min at room temperature for activation. 200 µg of coupling protein (BSA/KLH) was added and fixed on the rotator to be evenly mixed for 60 min at room temperature (the protein was dissolved with the polylink coupling buffer until 1-5 mg/mL). The coupling protein (BSA/KLH) was centrifuged for 10 min at 500-1000 g, the supernatant was sucked away, and then the amount of the coupling protein (BSA/KLH) was measured. 0.4 mL of polylink wash/storage buffer was added for resuspension, and the precipitate at the bottom of the tube was PS-BSA or PS-KLH and finally stored at 4°C. SDS-PAGE analysis (4% spacer gel and 7.5% separating gel) was performed. The detection results of reduced SDS-PAGE are shown in FIG. 1, proving that PS-KLH is successfully coupled with PS-BSA.
(2) Preparation of monoclonal antibody: 6 to 8-week-old female Balb/c mice were selected and immunologically injected with a prepared PS-BSA artificial antigen. Adult Balb/c mice received primary immunization through subcutaneous (in an area between shoulders) administration and were subjected to booster immunization every 2 weeks. Blood samples were collected at week 0 (before immunization), week 8 (2 weeks after the fourth booster jab), week 10 (before the third booster jab), week 12, and week 14, then serum isolation was performed to obtain B lymphocytes, and ribose nucleic acid (RNA) was extracted and transcripted into a complementary deoxyribonucleic acid (cDNA) library. Amplification was performed again through cDNA to obtain a specific gene fragment, and the specific gene fragment was recombined onto a bacteriophage to achieve the construction of a nano antibody gene library. Subsequently, specific antibody screening was performed to obtain an antibody meeting requirements, and the PS-BSA artificial antigen was fixed on a carrier and then interacted with the bacteriophage library. Then, the surface of the carrier was washed to remove unbound or non-specific bound bacteriophages, and then their binding was destroyed with a strong acid, thereby obtaining a positive bacteriophage solution. After 2-3 rounds were repeated, the monoclonal antibody meeting the requirements was obtained.

Through the injection of the PS-BSA artificial antigen, an immunization scheme for mice is shown in Table 1, the specific response of the PS-BSA artificial antigen on the immune system of Balb/c mice was observed, and an activation mechanism of an antibody-specific immune response was explored.

**Table 1 Immunization scheme for mice**

| Method | Antigen | Route | Adjuvant | Dose (µg) | Interval time distanced from the last immunization |
|---|---|---|---|---|---|
| The first immunization | PS-BSA | Subcutaneous | Complete | 100 µg/mouse | 0 |
| The second immunization | PS-BSA | Subcutaneous | Incomplete | 50 µg/mouse | 2 weeks |
| The third immunization | PS-BSA | Subcutaneous | Incomplete | 50 µg/mouse | 2 weeks |
| The fourth immunization | PS-BSA | Subcutaneous | Incomplete | 50 µg/mouse | 2 weeks |
| The fifth immunization | PS-BSA | Subcutaneous | Incomplete | 50 µg/mouse | 2 weeks |
| Enzyme-linked immunosorbent assay (ELLSA) tail blood potency testing | | | | | |
| Shock immunization | | Intraperitoneal, tail vein or intrasplenic | -- | 50 | 3 days before the fusion |

The monoclonal antibody comprises a heavy chain variable region and a light chain variable region both of which are composed of determining cluster complementarity regions and framework regions; the determining cluster complementarity regions of the heavy chain variable region and the light chain variable region are both composed of enzyme-linked immunosorbent assay (CDR1), CDR2 and CDR3;
The amino acid sequence of CDR1 of the heavy chain variable region is as shown in position 50-54 of SEQ ID NO.1;
The amino acid sequence of CDR2 of the heavy chain variable region is as shown in position 69-85 of SEQ ID NO.1;
The amino acid sequence of CDR3 of the heavy chain variable region is as shown in position 118-127 of SEQ ID NO.1;
The amino acid sequence of CDR1 of the light chain variable region is as shown in position 44-54 of SEQ ID NO.2;
The amino acid sequence of CDR2 of the light chain variable region is as shown in position 70-76 of SEQ ID NO.2;
The amino acid sequence of CDR3 of the light chain variable region is as shown in position 109-117 of SEQ ID NO.2.
The amino acid sequence of the heavy chain variable region of the antibody is as shown in SEQ ID NO.1: 
MEWSWIFLFLLSGTAGVHSEVQLQQSGPELVKPGASVKMSCKASGYTFTSYVM
HWVKQKPGQGLEWIGYINPYNDGTKYNEKFKGKATLTSDKSSSTAYMELSSLTSEDSA
 VYYCASSYYYGSSYGYWGQGTTLTVSS.
The amino acid sequence of the light chain variable region of the antibody is as shown in SEQ ID NO.2:

MESQIQVFVFVFLWLSGVDGDIVMTQSHKFMSTSVGDRVSITCKASQDVSTAVA
WYQQKPGQSPKLLIYSASYRYTGVPDRFTGSGSGTDFTFTISSVQAEDLAVYYCQQHYS
TPLTFGAGTKLELK.

Finally, it should be noted that the above descriptions are only preferred embodiments of the present disclosure, but are not used for limiting the present disclosure. Although the present disclosure has been illustrated in detail by referring to the foregoing embodiments, those skilled in the art still make modifications to the technical solutions described in the foregoing embodiments, or equivalent replacements on a part of technical features. Any modifications, equivalent replacements, improvements, and the like made within the spirit and principle of the present disclosure should be included within the scope of protection of the present disclosure.

## Claims

1. A one-step precipitation and color development method of a nitrocellulose (NC) membrane for micro/nano plastics, comprising the following steps:
S1, coupling micro/nano plastics with ovalbumin (OVA) to prepare a micro/nano plastic-OVA conjugate;
S2, streaking the micro/nano plastic-OVA conjugate on an NC membrane to prepare an NC membrane coated with the micro/nano plastic-OVA conjugate;
S3, putting the NC membrane coated with the micro/nano plastic-OVA conjugate into a polystyrene antibody solution and a sample solution to be incubated for 1 h at 37°C, and then rinsing the NC membrane once with phosphate-buffered saline Tween (PBST) and taking out the rinsed NC membrane; wherein the polystyrene antibody is secreted by hybridoma cell line polystyrene (PS)-8 which is deposited with China Center for Type Culture Collection under the Accession number of CCTCC NO: C2024144 on May 10, 2024, and the address of the collection unit is 299 Bayi Road, Wuchang District, Wuhan City, Hubei Province, on campus of Wuhan University;
S4, diluting goat anti-mouse lgG-horseradish peroxidase (HRP) with 20% N-bromosuccinimide-phosphate buffer saline (NBS-PBS) by 1w fold and then putting the diluted goat anti-mouse IgG-HRP in a centrifuge tube, putting the NC membrane into the centrifuge tube, and then incubating for 30 min at 37°C; and
S5, washing the NC membrane 4-5 times and sucking away water, completely immersing the NC membrane into precipitating tetramethylbenzidine (TMB) for 30 s and then taking out the NC membrane and sucking away water, and placing the NC membrane at room temperature for color development, followed by detecting the concentration of micro/nano plastics.

2. The one-step precipitation and color development method of the NC membrane for micro/nano plastics according to claim 1, wherein in step S1, the coupling the micro/nano plastics with OVA comprises the following steps:
S11, cooling the microplastics, polylinker coupling buffer, and polylinker wash/storage buffer to room temperature;
S12, putting the microplastics into a centrifuge tube, sucking away the supernatant after centrifuging, adding the polylinker coupling buffer for resuspension, sucking away the supernatant after centrifuging again, and resuspending with the polylinker coupling buffer;
S13, dissolving polylinker ethyl-[3-(dimethylamino)propyl]-carbodiimide hydrochloride (EDAC) into the polylinker coupling buffer to prepare an EDAC solution;
S14, adding the EDAC solution into the microplastic suspension, and then evenly mixing the above materials for activation;
S15, adding an OVA protein, diluting the OVA protein into 1-5 mg/mL with the polylinker coupling buffer using the polylinker coupling buffer, and evenly mixing the above materials for 30-60 min under the condition of rotating at room temperature;
S16, sucking away the supernatant after centrifuging;
S17, adding 0.4 ml of polylinker coupling buffer for resuspension; and
S18, repeating steps S16 and S17 to finally obtain the micro/nano plastic-OVA conjugate, and then storing the conjugate at 4°C.

3. The one-step precipitation and color development method of the NC membrane for micro/nano plastics according to claim 1, wherein in step S5, the detecting the concentration of the micro/nano plastics comprises the following steps:
S51, photographing the NC membrane to obtain the color developed with precipitating TMB;
S52, transforming the color developed with the precipitating TMB into a gray value and then establishing a standard curve with the transformed gray value and the concentration; and
S53, calculating the concentration of the micro/nano plastics in the to-be-detected sample by inputting the gray value of a to-be-detected sample into the standard curve.

## Amended claims

### Amended claims in accordance with Rule 137(2) EPC.

1. A precipitation and color development method of a nitrocellulose (NC) membrane for micro/nano plastics, comprising the following steps:
S1, coupling micro/nano plastics with ovalbumin (OVA) to prepare a micro/nano plastic-OVA conjugate, wherein the micro/nano plastic derived from polystyrene plastic;
S2, streaking the micro/nano plastic-OVA conjugate on an NC membrane to prepare an NC membrane coated with the micro/nano plastic-OVA conjugate;
S3, putting the NC membrane coated with the micro/nano plastic-OVA conjugate into a polystyrene antibody solution and a sample solution to be incubated for 1 h at 37°C, wherein the micro/nano plastic in the sample solution competes with the PS-OVA conjugate on the NC membrane for binding to the PS antibody; and then rinsing the NC membrane once with phosphate-buffered saline Tween (PBST) and taking out the rinsed NC membrane; wherein the polystyrene antibody is secreted by hybridoma cell line polystyrene (PS)-8 which is deposited with China Center for Type Culture Collection under the Accession number of CCTCC NO: C2024144 on May 10, 2024, and the address of the collection unit is 299 Bayi Road, Wuchang District, Wuhan City, Hubei Province, on campus of Wuhan University, wherein the sample solution is PS sample extracting dilution solution;
S4, diluting goat anti-mouse IgG-horseradish peroxidase (HRP) with 20% N-bromosuccinimide-phosphate buffer saline (NBS-PBS) by 10000 fold and then putting the diluted goat anti-mouse IgG-HRP in a centrifuge tube, putting the NC membrane into the centrifuge tube, and then incubating for 30 min at 37°C; and
S5, washing the NC membrane 4-5 times and sucking away water, completely immersing the NC membrane into precipitating tetramethylbenzidine (TMB) for 30 s and then taking out the NC membrane and sucking away water, and placing the NC membrane at room temperature for color development, followed by detecting the concentration of micro/nano plastics.

2. The precipitation and color development method of the NC membrane for micro/nano plastics according to claim 1, wherein in step S1, the coupling of the micro/nano plastics with OVA comprises the following steps:
S11, cooling the microplastics, polylinker coupling buffer, and polylinker wash/storage buffer to room temperature;
S12, putting the microplastics into a centrifuge tube, sucking away the supernatant after centrifuging, adding the polylinker coupling buffer for resuspension, sucking away the supernatant after centrifuging again, and resuspending with the polylinker coupling buffer;
S13, dissolving polylinker ethyl-[3-(dimethylamino)propyl]-carbodiimide hydrochloride (EDAC) into the polylinker coupling buffer to prepare an EDAC solution;
S14, adding the EDAC solution into the microplastic suspension, and then evenly mixing the above materials for activation;
S15, adding an OVA protein, diluting the OVA protein into 1-5 mg/mL with the polylinker coupling buffer using the polylinker coupling buffer, and evenly mixing the above materials for 30-60 min under the condition of rotating at room temperature;
S16, sucking away the supernatant after centrifuging;
S17, adding 0.4 ml of polylinker coupling buffer for resuspension; and
S18, repeating steps S16 and S17 to finally obtain the micro/nano plastic-OVA conjugate, and then storing the conjugate at 4°C.

3. The precipitation and color development method of the NC membrane for micro/nano plastics according to claim 1, wherein in step S5, detecting the concentration of the micro/nano plastics comprises the following steps:
S51, photographing the NC membrane to obtain the color developed with precipitating TMB;
S52, transforming the color developed with the precipitating TMB into a gray value and then establishing a standard curve with the transformed gray value and the concentration; and
S53, calculating the concentration of the micro/nano plastics in the to-be-detected sample by inputting the gray value of a to-be-detected sample into the standard curve.
